# EUROPEAN PATENT APPLICATION

(11) **EP 4 610 649 A1**
(43) Date of publication of application: **03.09.2025**
(21) Application number: 24160821.5
(22) Date of filing: 01.03.2024
(51) Int. Cl.: G01N 33/205

(54) **SAMPLING SYSTEM AND METHOD FOR TAKING SAMPLES FROM MOLTEN ALUMINUM IN A PRIMARY ALUMINUM PRODUCING FACILITY**

(71) Applicant: Heraeus Electro-Nite International N.V., 3530 Houthalen (BE)
(72) Inventor: Kendall, Martin, 3530 Houthalen (BE); Zels, Adriaan, 3530 Houthalen (BE); Cappa, Chris, 3530 Houthalen (BE)
(74) Representative: Heraeus IP

(57) **Abstract**

The present invention relates to a sampling system comprising a lance and a sample unit for taking samples from a molten aluminum bath in a primary aluminum producing facility. The sample unit is configured to receive a sample of molten aluminum, comprising an inlet opening and a sample chamber in flow connection with the inlet opening and the lance is configured to engage the sample unit. The immersion end of the lance is made from a non-magnetic material with a melting point higher than 950 °C. The invention further relates to a lance, a sample unit and a method for taking samples from molten aluminum.

## Description

The present invention relates to a sampling system comprising a lance and a sample unit for taking samples from a molten aluminum bath in a primary aluminum producing facility. The invention further relates to a lance, a sample unit and a method for taking samples from molten aluminum.

During the processing of metals in their molten state, it is necessary to obtain a representative sample of the molten metal at various stages of the process, for example, for the analysis or evaluation of either the chemical composition or the metallographic structure of the sample. Different methods for analyzing molten metals during manufacturing and further processing are known in the art.

Historically, the composition of a solidified metal sample is often determined using arc spark-optical emission spectroscopy (spark-OES). Spark-OES systems are effective systems for determining the chemical composition of a metal sample and for controlling the processing of molten metals due to their rapid analysis times and inherent accuracy. Recently, also LIBS spectroscopy has been applied to obtain the data of interest.

Irrespective of the type of analysis, the accuracy and reliability of the obtainable results has been known to be dependent on the quality of the sample. It is desired that the taken sample can be analyzed without any further preparation steps and as close to the place of sampling as possible.

Primary aluminum (Al) production is to be distinguished from secondary aluminum production: primary aluminum is produced from Al raw material, typically bauxite, which is chemically treated to obtain alumina, wherein secondary Al is produced from recycled Al scrap or from primary aluminum which is further treated, alloyed and/or re-molten. For secondary aluminum production, the scrap is molten in a suitable furnace and processed and treated further as needed.

Primary aluminum is typically produced by the Hall-Héroult process. This electrolytical process is based on the electrochemical decomposition of alumina dissolved in a sodium-cryolite electrolyte (Na₃AlF₆) at 900 - 1000 °C. The obtained aluminum composition is depending on the composition of the incoming materials, the process conditions and the state of the process. In metallurgical facilities producing aluminum, pots containing the refined material in different stages are tapped daily or every second day. The batches of different pots will be mixed in the cast house to produce the targeted grades. Some elements are known to be critical for the quality of the final product and thus their levels need to be determined before the blending starts.

The pots of a primary aluminum producing facility are arranged side to side, whereby each pot contains around 10 t of liquid aluminum underneath a layer of liquid and solidified salts (the cryolite layer). The liquid metal bath depth and height will vary with the quantity of aluminum and the age of the pot. Due to the geometry of these pots, the sampling cannot be done with visual contact to the metal interface.

In order to obtain an aluminum sample from a pot the state of the art sampling procedure comprises the utilization of a preheated coated spoon as a sampling device. The spoon is inserted through the cryolite in the aluminum pool and after retracting the filled spoon, the cryolite is poured back and the remaining aluminum is poured in a copper mold. After a cooling period, the sample is obtained and sent to a remote lab, where it is prepared to be suitable for a final analysis. These steps are time consuming, and it is desired to shorten the time between sampling and the final analysis. Furthermore, the manual handling of the molten aluminum with a spoon is a dangerous operation which shall be avoided.

Sampling systems comprising a lance which carries a suitable sample unit are commonly applied in iron and steel foundry applications. Such devices are for example disclosed in EP 0893681 A1. These devices are used to immerse the sample unit under the surface of the molten metal to be sampled, where a sample chamber positioned in the sample unit is filled. Between lance and sample body, a cardboard tube can be arranged to extend the length of the whole device and thus avoid the contact between the molten metal and the lance. The sample units typically comprise two parts: a refractory body and a sample chamber assembly which is arranged within the refractory body. The sample chamber assembly comprises molds which delineate the hollow sample chamber, typically these molds are either a two-part clam shell type arrangement or a ring covered on its upper and lower sides by flat plates. The sample unit further has a suitable opening through which the molten metal can enter the sample chamber. After sampling, the whole device is retracted from the molten metal and the refractory body is removed from the sample chamber assembly after solidification of the obtained sample. The remaining parts of the refractory body and sample chamber assembly are waste and need to be disposed. A conventional material for the re-usable lance and the sample chamber assembly is steel, while the refractory body typically comprises a sand material, like resin coated silica-based sand. While these material selections are suitable for steel producing facilities, they cannot be applied in aluminum foundries, since the iron (Fe) and silicon (Si) contained in the steel parts, or the sand are not desired in the final aluminum product. For this reason, they can also not be disposed in the molten aluminum bath.

The utilization of this technique in aluminum sampling applications is not trivial and has for further reasons not been disclosed in the prior art: The circumstances of sampling are not comparable - in steel facilities a direct view on the sampling place is possible, while in primary aluminum pots such a direct control is hindered. Furthermore, the cryolite layer covering the primary aluminum bath poses additional demands on the sampling system - the sampling device needs to be capable to travel through the cryolite layer without a penetration of the salts into the sample chamber. Furthermore, the materials applied in the state of the art systems are not compatible with the demands of an Al producing facility.

For the above explained reasons, it is desired to provide a sampling system which can be applied to obtain samples from aluminum, especially in primary aluminum producing facilities.

An objective of the present invention was to provide such a sampling system.

It was a further objective to provide a sampling system, of which all used materials are compatible with the aluminum producing process to obtain a waste-free system. Especially the use of steel components, silica-based sand and paper-based components should be avoided.

Furthermore, all single-use parts should be suitable to be disposed in the aluminum melt without introducing contaminating elements.

A further objective was to provide a sample unit for the sample system. In an additional aspect, the sample unit should be compatible with the demands and environment of an aluminum producing facility, in particular it should minimize the contamination introduced with the sample unit.

In a further aspect, it was an objective of the invention to provide a lance for a sampling system to obtain samples from primary aluminum. In an additional aspect, the lance should be re-usable and compatible with the demands and environment of a primary aluminum producing facility. In a further aspect, the lance should be immersible in the molten aluminum bath, especially in an aluminum bath in a primary aluminum producing facility.

A further objective was to provide a method for taking samples from molten aluminum with a sampling system comprising a lance and a sample unit.

These objectives are attained by the subject-matter defined in the independent claims.

The invention provides a sampling system for taking samples from a molten aluminum bath in a primary aluminum producing facility, comprising
- a lance, having an immersion end and a handling end,
- a sample unit configured to receive a sample of molten aluminum, comprising an inlet opening and a sample chamber in flow connection with the inlet opening,
wherein the lance is configured to engage the sample unit,
characterized in that
the immersion end of the lance is made from a non-magnetic material with a melting point higher than 950 °C.

Preferred embodiments are defined in the dependent claims. The preferred embodiments may be realized individually or in any possible combination.

The present invention provides a combination of a sampling unit, that minimizes the return of waste materials and delivers an easy and directly to analyze sample, in combination with an immersion lance avoiding all risks of bath contamination. The immersion lance is reusable and maintenance free. Surprisingly, it has been found that such a sampling system can be utilized to obtain samples from molten aluminum, especially from molten aluminum in a primary aluminum producing facility.

Advantageously, the inventive sampling system comprises a minimal number of components. Especially, additional carrier means like paper tubes can be avoided.

An additional benefit of this sampling system is the combability with the environment present in an aluminum producing facility.

The invention relates to a sampling system for taking samples from a molten aluminum bath.

As used herein, the term "molten aluminum bath" is used to describe a melt of aluminum in a vessel. An alternative term for "molten aluminum bath" known to a skilled person is "aluminum melt". The term molten aluminum bath does not exclude the presence of any solid or gaseous parts, including for example non-molten parts of the respective metal or solid raw materials. In primary aluminum producing facilities, the molten aluminum bath may be covered with a cryolite layer. Additionally, the aluminum bath may be covered with a crust of solid material which is positioned above the cryolite layer, in other words, the cryolite layer may be positioned between such a crust and the aluminum melt. It is to be understood, that the crust also comprises cryolite.

The sampling system is especially advantageous for taking samples in primary aluminum producing facilities. Primary aluminum is to be understood as aluminum produced by electrolytic processing of aluminum containing raw materials like alumina.

The temperature of aluminum melts differs and usually depends on the composition of the aluminum and the stage of the production process. According to a preferred embodiment, the temperature of the molten aluminum bath is in the range of 800 - 1100 °C and more preferably in the range of 850 - 1000 °C.

The sampling system comprises a lance, which has an immersion end and a handling end.

The immersion end is to be understood as the end portion of the lance on one side. The immersion end is configured to be immersible in the molten aluminum. The immersion end ends in an immersion tip, in other words, the end portion of the immersion end is the immersion tip. The handling end is the opposite end of the lance, in other words, the lance extends from the immersion end to the handling end.

The lance can be formed as a straight pole, it may also be bent. Preferably, the lance is bent. Such a construction allows the lance to have at least two portions: an immersion portion extending from the immersion end to the bend and a handling porting extending from the bend to the handling end. Preferably, the bent angle is in the range between 120 - 160 ° in such cases.

The lance can be formed as a monolithic device, it can also comprise a number of parts which are fixated to each other in a suitable way, for example by threading means, welding, press-fitting or combinations thereof. The lance may comprise additional parts, like means for handling, which are preferably foreseen at the handling end of the lance.

The material of the lance is selected to be compatible with the environment of the aluminum producing facility. For example, the lance and especially the immersion part needs to be resistant to high temperatures, in particular resistant against the temperature of the molten aluminum. The melting temperature of aluminum is 660 °C, however, the temperature of the molten aluminum bath in an aluminum producing facility is typically significantly higher. Such a temperature resistance may be particularly relevant to portions of the lance which are immersible. Thus, the material of the immersion end of the lance has a melting point higher than 950 °C.

Furthermore, the material may be resistant against oxidization, even at elevated temperatures. For similar reasons, the lance may need to be resistant to corrosion by the molten aluminum and/or cryolite. Corrosion resistance may be useful or requisite for other reasons too, including prolonging the lifespan of the lance. In this context, "corrosion" is to be interpreted broadly, including any one or more of corrosion, dissolution, erosion, chemical reactivity, or any process that may damage or destroy the lance. Corrosion resistance may also ensure that the lance can be repeatedly utilized, to reduce the expense associated with the sampling system and/or the lance and associated waste production. Such corrosion resistance may be particularly relevant to portions of the lance which are immersible.

In order to be usable in the environment of an aluminum producing facility, in particular a primary aluminum producing facility, no magnetic parts shall be applied in the vicinity of the vessels comprising the molten aluminum. Thus, the immersion end of the lance is made from a non-magnetic material. The non-magnetic material is to be understood as a material which is not attracted or repelled by a magnet, in other words, the material has a very low or negligible response to a magnetic or electromagnetic field. Preferably, the relative magnetic permeability of the non-magnetic material is lower than 1,3, more preferred lower than 1,2, most preferred lower than 1,1. The relative magnetic permeability is defined as the ratio of the absolute magnetic permeability to the value of the magnetic permeability of vacuum. Non-magnetic materials are defined as materials which have a low relative magnetic permeability. Materials commonly referred to as "magnetic" (ferromagnetic and ferrimagnetic) have a significantly higher relative magnetic permeability.

Preferably, all parts of the lance are made from a non-magnetic material.

Preferably, the material of the immersion end of the lance has a heat capacity of at least 100 J/(kg*K), more preferred of at least 200 J/(kg*K), even more preferred of at least 300 J/(kg*K . For example, the material may have a heat capacity in the range of 100 to 600 J/(kg*K), more preferred in the range of 200 to 500 J/(kg*K).

Suitable materials for the immersion end of the lance are for example stainless steel and copper. In preferred embodiments, the immersion end of the lance is made from stainless steel, in particular from austenitic stainless steel, for example stainless steel 316L.

The lance may comprise only one material, it can also be advantageous when different parts of the lance comprise different materials. For example, the immersion end may be made from stainless steel, while the handling end can be made from aluminum. Thus, the lance assembly can be designed cost effective and light weight.

The immersion end of the lance is preferably formed as a hollow body, for example as a tube. The cross-section of the immersion end can have a circular shape, it may also have various other shapes (e.g. square, triangular, polygonal). The hollow body, in particular a tube, can have a thickness in the range of 1 to 10 mm, with an internal diameter of between 5 and 35 mm and an external diameter between 10 to 40 mm. The tube can be foreseen with coupling means configured to couple several parts of the lance to each other, for example by threading means.

The lance is configured to engage the sample unit, preferably it is configured to be inserted into the sample unit. Most preferably, the immersion end of the lance is configured to be inserted into the sample unit. In other words, the immersion end of the lance carries the sample unit in use. In particular, the immersion tip is configured to engage the sample unit, in other words, the immersion tip is configured to be inserted into the sample unit. The external diameter and shape of the immersion tip is configured to mate with the sampling unit, especially to fit flush in the sample unit. In such cases, the sample unit is configured to be engaged by the immersion end of the lance, preferably the sample unit comprises an indentation which is configured for the immersion end of the lance to be inserted.

Preferably, the immersion end of the lance, in particular the immersion tip, has a tapered section, the tapered section being a section comprising a cross-section which is tapered in a longitudinal direction towards the tip of the immersion end. If not otherwise defined, the cross-sectional area is the cross-sectional area perpendicular to the longitudinal axis of the lance along its length. The immersion end, in particular the immersion tip, preferably has a frusto-conical-shape. Preferably, the diameter of the immersion end tapers by not more than 10% towards the immersion tip. The diameter of the immersion end may taper from a diameter between 10 to 40 mm towards a diameter between 5 to 38 mm. Such a shape allows the immersion lance to be press-fitted in the sample unit in an efficient way. In such cases, it is in particular advantageous when the sample unit comprises an indentation with a cylindrical geometry with an inclining sidewall towards its base. In other words, the indentation of the sample unit preferably has a larger diameter on the side where the lance is inserted and a smaller diameter on its base side. It can analogously be advantageous that the internal diameter of the indentation of the sample unit tapers from a diameter between 10 to 40 mm towards a base diameter between 5 to 38 mm. Preferably, the inner diameter of the indentation of the sample unit tapers by not more than 10 % towards the base.

The immersion end may be part of the lance-body, it may also be provided as an additional part, in particular as an immersion tip. In preferred embodiments, the immersion tip of the lance is detachable. Thus, the immersion tip, which is the part of the lance which is exposed to the most wear in use, can be easily exchanged. The immersion tip may be fixated to the other parts of the lance by suitable means, for example by threading means of welding. The immersion tip can be foreseen with coupling means configured to couple the immersion tip to the adjacent part of the lance, for example by threading means. The immersion tip and the adjacent part of the lance thus may have respective internal and external diameters to enable the coupling. In other embodiments, the immersion tip and the adjacent part of the lance may have similar diameters, with their ends abutting flush to one another being joined by additional coupling means or a weld.

In preferred embodiments, the lance comprises protrusions located at the immersion end, preferably at the immersion tip of the lance. A protrusion is to be understood as a singular item that projects out of a surface. Such protrusions allow an easy but safe fixation of the sample unit. The protrusions may be part of the immersion end or the immersion tip, in other words the immersion end and the protrusions may be monolithically formed. The protrusions may also be provided as additional components. For example, the immersion end may be tube shaped and comprise a plurality of radial bores in the lateral surface of the tube. Protruding elements can be fixed in the bores, for example by threading means or welding. When the immersion end comprises a tapered section, the protrusions are preferably positioned within this tapered section.

The shape of the protrusions is not further restricted, they may for example be pyramidal, cylindrical, conical or frusto-conical shaped.

Preferably, the protrusions are provided in such a way that they cannot be pushed back when the immersion end is inserted into the sample unit, in particular, the protrusions are not springloaded. In preferred embodiments, the protrusions have a greater hardness than the face of the sample unit with which they engage.

The number of protrusions is not restricted, for example, the lance may comprise two, three, four, five, six or seven protrusions. Preferably, the protrusions are evenly distributed around the circumference of the immersion end.

In preferred embodiments, the lance comprises a collar at the immersion end. A collar is to be understood as a part which has a larger diameter than the rest of the lance and is arranged in such a way, that it protrudes from the axial length of the lance. Preferably, the collar is part of the immersion tip. In embodiments comprising protrusions, the collar is preferably positioned above the protrusions in a direction from the immersion end to the handling end.

Preferably, the collar is configured to sit flush on the sample unit. It has surprisingly been found that the provision of a collar on the lance, which is directly placed on the sample unit, improves the usability of such a sampling system for aluminum applications.

Preferably the outer diameter of the collar is at least 20 % larger than the outer diameter of the immersion end of the lance, more preferred at least 30 % larger, even more preferred at least 40 % larger. In particular, the outer diameter of the collar should not exceed the diameter of the face of the sample unit on which it is arranged. The outer diameter of the collar is to be understood as the largest diameter of the collar perpendicular to the longitudinal axis of the lance, wherein the longitudinal axis is an axis extending between the immersion end to the handling end. In cases where the lance comprises a bend or a kink, the longitudinal axis is the axis extending on a straight portion between the immersion end to the bend.

Preferably, the side of the collar facing the immersion end of the lance is flat. Thus, the flat face forms a sealing face towards the sample unit when the lance is pressed in and on the sample unit. Further sealing means are not required which further improves the simple design of the sampling system and reduces the use of potentially contaminating materials.

The collar can have an inclined shaped face, in particular a conical shaped face, opposite the flat face. The collar may be ring shaped, it may also be frusto-conical shaped or a combination of these shapes.

The collar may be part of the immersion end, in other words the immersion end and the collar may be monolithically formed. The collar may also be provided as a separate component. In such cases, the collar may be part of the immersion tip of the lance which may also comprise protrusions.

The material of the collar may be the same or different as the material of the other components of the lance. Preferably, the collar is made from the same material as the immersion end and/or the immersion tip.

The lance may comprise additional parts arranged within a hollow body structure at the immersion end. Preferably, the lance comprises an inner tube arranged at the immersion end inside a hollow body structure of the lance. Such a tube can provide a ventilation path away from the sample unit when the unit is filled during sampling and allows to obtain samples with a higher quality.

The inner tube is preferably configured to be fixed inside the hollow body structure of the lance by suitable means, for example by threading means or welding.

The sampling system comprises a sample unit configured to receive a sample of molten aluminum. It is to be understood, that the sample unit is a disposable item, while the lance is reusable.

The sample unit comprises a top side and a bottom side. The top side of the sample unit is configured to be engaged by the immersion end of the lance, preferably in such a way that the immersion end of the lance can be inserted into the sample unit. Preferably, the top side is configured to completely surround the immersion tip of the lance. In preferred embodiments, the top side comprises an indentation configured to interact with the immersion end of the lance. Preferably, the indentation is cylindrical or frusto-conical shaped.

When the immersion end of the lance is inserted into the indentation of the sample unit, protrusions positioned at the immersion end of the lance can engage with the body of the sample unit. Such a configuration allows the fixation of the sample unit on the lance without the need for any further fixation means or adhesives.

Exemplary sample units can have a height between 20 and 80 mm, preferably between 30 und 70 mm. Preferably, the diameter of the sample unit is in the range between 20 and 80 mm, preferably between 30 und 70 mm.

In advantageous embodiments, the mass of the sample unit is lower than 150 g, preferably lower than 120 g, most preferred lower than 100 g. In steel applications, the weight of commonly used sample units is around 300 g, the sample unit according to the invention has thus a much smaller weight, which additionally enhances its usability for aluminum sampling applications.

The sample unit comprises an inlet opening and a sample chamber in flow connection with the inlet opening. A sample chamber is to be understood as a hollow part inside the sample unit which is configured to receive the molten aluminum.

Preferably, the inlet opening is positioned in the face extending between the top side and the bottom side of the sample unit. The inlet opening may be protected with suitable means to ensure that cryolite cannot enter the sample unit before the sample unit reaches the sampling position, for example the inlet opening may be covered with a material that dissolves or burns once it is exposed to the environment of the molten aluminum. Suitable means can for example be a tape or a plug, for example a paper tape.

The sample unit can comprise an inflow conduit configured to be received by the inlet opening. It is to be understood that such an inflow conduit establishes a flow connection between the inlet opening and the sample chamber. An inflow conduit thus enables the flow of molten metal from a molten aluminum bath into the sample chamber. The inflow conduit may comprise an inlet opening extending out of the sample unit or ending at the outside of the sample unit and an outlet opening extending in the sample chamber or ending at the sample chamber. The inflow conduit is preferably made of a quartz material, more preferably a fused quartz material. However, it will be understood that the inflow conduit may be made of any other suitable material, including, but not limited to, a ceramic or metal material, for example stainless steel.

Preferably, the sample chamber has a circular cross-section perpendicular to the longitudinal axis of the sample unit. The longitudinal axis is to be understood as the axis connecting the top side and the bottom side. Also preferred is a sample chamber which comprises two flat faces opposite one another along the longitudinal axis. In an advantageous embodiment of the invention, the sample chamber is constructed cylindrically, and the base surface of the sample chamber has an approximately circular cross section, whose diameter is greater than the thickness of the sample chamber along the longitudinal axis. In other words, the sample chamber is preferably disc shaped. Disc shaped samples are in particular advantageous because they can be easily directly analyzed without further sample preparation.

The inlet opening is advantageously connected to a side face of the sample chamber, in other words, the inlet opening is positioned in a face of the sample chamber which is not aligned on the longitudinal axis of the sample unit.

In preferred embodiments, the sample unit comprises a refractory body and a sample chamber assembly arranged within the refractory body. The sample chamber assembly is configured to receive a sample of molten aluminum when the sampling system is immersed in a molten aluminum bath.

The refractory body may have a cylindrical or barrel shape. It is to be understood, that the refractory body builds the outer part of the sample unit. A suitable refractory body can have a height between 20 and 80 mm, preferably between 30 und 70 mm. Preferably, the diameter of the refractory body is in the range between 20 and 80 mm, preferably between 30 und 70 mm.

The refractory body may be a monolithic body it may also comprise a plurality of parts. For example, the refractory body may comprise an essentially tube-shaped part and a closing means closing one of the openings of the tube. The whole refractory body can be a molded sand body or the plurality of parts building it can be molded sand bodies.

It is to be understood, that the refractory body comprises an inlet opening in flow connection with the sample chamber.

The refractory body may be made from any material which is suitable to withstand the sampling environment, preferably the material is also compatible with the demands of an aluminum producing facility, in particular with the demands of a primary aluminum producing facility. Preferably, the refractory material is a sand material, for example a resin coated sand material. In especially advantageous embodiments, the refractory material has a silica (silicon oxide) content lower than 80 mass.-%, more preferred lower than 70 mass.-%., even more preferred lower than 60 mass.-%, even more preferred lower than 50 mass.-%, most preferred the refractory material is silicon (Si) free apart from unavoidable impurities. The mass.-% of silica is to be understood of the mass percentage of silica in the total weight of the refractory material.

Preferably, the refractory material has a content of iron oxides (FeOₓ) lower than 5 mass.-%., more preferred lower than 3 mass.-%., even more preferred lower than 1 mass.-%, most preferred the refractory material is iron (Fe) free apart from unavoidable impurities. The mass.- % of iron oxides is to be understood of the mass percentage of iron oxides in the total weight of the refractory material.

The refractory material may comprise a binder material, for example a phenolic resin. Preferably, the refractory material comprises 1-8 mass.-% of a binder material, more preferred 3-5 mass.-%. The mass.-% of binder material is to be understood of the mass percentage of binder material in the total weight of the refractory material.

A suitable refractory material can for example consist of 3 - 5 mass-% of a binder material, 0 - 50 mass.-% silica, impurities and/or other components of not more than 1 mass.-% and the rest to add up to 100 mass.-% of alumina.

Preferably, the refractory body has a certain porosity to permit the gases developed during the sampling operation to escape in a simple way through the porous structure. Preferably, the refractory body has a porosity of at least 20 %, more preferred of at least 30 %. For example, the refractory body may have a porosity in the range of 20 to 50 %, preferably in the range of 30 to 40 %. The porosity refers to the measure of the void spaces within the refractory body. It is to be understood as the volume of open spaces within the refractory body divided by the total volume of the refractory body.

Preferably, the sample chamber assembly is completely arranged within the refractory body. Thus, the refractory body comprises the top side and the bottom side of the sample unit. The top side is preferably configured to receive the lance. Preferably, the bottom part is closed. In other words, the side which first immersed into the molten aluminum during the sampling procedure does not comprise an opening.

Preferably, the sample chamber assembly comprises at least two parts, configured to be detachable assembled to form the sample chamber. In other words, the sample chamber assembly encloses the hollow volume of the sample unit which forms the sample chamber.

The sample chamber assembly may for example comprise two mold parts in form of half shells, in an alternative embodiment the sample chamber assembly may comprise two flat discs and a ring.

In preferred embodiments, the parts of the sample chamber assembly can be pressed against one another, and thus held together, by closing means, for example by a spring, a clip or a clamp. Alternatively, or additionally, fixation means can be foreseen, for example an adhesive attaching the parts of the sample chamber to each other. In advantageous embodiments, the sample chamber assembly is arranged in the refractory body in such a way, that no further closing means are needed. In other words, the sample chamber assembly is preferably clamped in the refractory body. By using such a configuration for holding the sample chamber assembly together, the parts are held together by compressional forces applied by the refractory body and no glues or cements are needed to be used for said closing. Thus, a simplified sample unit is obtained which further reduces the amount of potentially contaminating material being brought into contact with the molten aluminum to be sampled.

The sample chamber assembly is preferably formed of one or more materials which are good thermal and electrical conductors, such as, but not limited to, aluminum, copper and other metals having similar thermal and electrical conductivity properties. Preferably, the sample chamber assembly is made of aluminum. By employing aluminum, no additional contaminating elements are introduced into the molten aluminum during sampling and additionally, the remains of the sample chamber assembly after the sampling procedure can be directly disposed in the aluminum bath.

It is to be understood that the sample chamber assembly comprises an inlet opening which is in flow connection with the inlet opening of the refractory body. Thus, there is a fluid connection between the inlet opening of the sample unit and the sample chamber. Preferably, the assembled sample chamber assembly comprises only one opening. Preferably, the one opening is configured to receive an inflow conduit. Such an inflow conduit enhances the flow of molten aluminum from a molten aluminum bath into the sample chamber assembly.

Preferably, the parts of the sample chamber assembly are not arranged in a gas-tight manner, in other words the sample chamber assembly is gas permeable. Thus, gases present in the sample chamber can leave the sampling chamber while the molten metal flows in, so that gas inclusions in the sample are prevented.

The mass of the sample chamber assembly preferably accounts for not more than 40 % of the mass of the sample unit, preferably for not more than 30 %. For example, the mass of the sample chamber assembly may account to 10 to 40 % of the overall mass of the sample unit, more preferred to 20 to 30 %.

A further aspect of the invention is a sample unit for a sampling system according to the invention. All features, advantages and embodiments mentioned in relation to the sampling system according to the invention also apply to the sample unit of the invention.

A further aspect of the invention is a lance for a sampling system for taking samples from a molten aluminum bath in a primary aluminum producing facility, wherein the lance has an immersion end and a handling end, characterized in that the immersion end of the lance is made from a non-magnetic material with a melting point higher than 950 °C.

All features, advantages and embodiments mentioned in relation to the sampling system according to the invention also apply to the inventive lance.

Surprisingly, it has been found that a lance with the claimed features is suitable as a re-usable tool in the sampling of primary aluminum.

The immersion end is to be understood as the end portion of the lance on one side. The immersion end is configured to be immersible in the molten aluminum. The immersion end ends in an immersion tip, in other words, the end portion of the immersion end is the immersion tip. The handling end is the opposite end of the lance, in other words, the lance extends from the immersion end to the handling end.

The lance can be formed as a straight pole, it may also be bent. Preferably, the lance is bent. Such a construction allows the lance to have at least two portions: an immersion portion extending from the immersion end to the bend and a handling porting extending from the bend to the handling end. Preferably, the bend angle is in the range between 120 - 160 ° in such cases.

The lance can be formed as a monolithic device, it can also comprise a number of parts which are fixated to each other in a suitable way, for example by threading means, welding, press-fitting or combinations thereof. The lance may comprise additional parts, like means for handling, which are preferably foreseen at the handling end of the lance.

The material of the lance is selected to be compatible with the environment of the aluminum producing facility. For example, the lance and especially the immersion part needs to be resistant to high temperatures, in particular resistant against the temperature of the molten aluminum. The melting temperature of aluminum is 660 °C, however, the temperature of the molten aluminum bath in an aluminum producing facility is typically even higher. Such a temperature resistance may be particularly relevant to portions of the lance which are immersible. Thus, the material of the immersion end of the lance has a melting point higher than 950 °C.

Furthermore, the material may be resistant against oxidization, even at elevated temperatures.

For similar reasons, the lance may need to be resistant to corrosion by the molten aluminum and/or cryolite. Corrosion resistance may be useful or requisite for other reasons too, including prolonging the lifespan of the lance. In this context, "corrosion" is to be interpreted broadly, including any one or more of corrosion, dissolution, erosion, chemical reactivity, or any process that may damage or destroy the lance. Corrosion resistance may also ensure that the lance can be repeatedly utilized, to reduce the expense associated with the sampling system and/or the lance and associated waste production. Such corrosion resistance may be particularly relevant to portions of the lance which are immersible.

In order to be usable in the environment of an aluminum producing facility, in particular a primary aluminum producing facility, no magnetic parts shall be applied in the vicinity of the vessels comprising the molten aluminum. Thus, the immersion end of the lance is made from a non-magnetic material. Preferably, all parts of the lance are made from a non-magnetic material.

Preferably, the material of the immersion end of the lance has a heat capacity of at least 100 J/(kg*K), more preferred of at least 200 J/(kg*K), even more preferred of at least 300 J/(kg*K . For example, the material may have a heat capacity in the range of 100 to 600 J/(kg*K), more preferred in the range of 200 to 500 J/(kg*K).

Suitable materials for the immersion end of the lance are for example stainless steel and copper. In preferred embodiments, the immersion part of the lance is made from stainless steel, in particular from austenitic stainless steel, for example stainless steel 316L.

The lance may comprise only one material, it can also be advantageous when different parts of the lance comprise different materials. For example, the immersion end may be made from stainless steel, while the handling end can be made from aluminum. Thus, the lance assembly can be designed cost effective and light weight.

The immersion end of the lance is preferably formed as a hollow body, for example as a tube. The cross-section of the immersion end can have a circular shape, it may also have various other shapes (e.g. square, triangular, polygonal). The hollow body, in particular a tube, can have a thickness in the range of 1 to 10 mm, with an internal diameter of between 5 and 35 mm and an external diameter between 10 to 40 mm. The tube can be foreseen with coupling means configured to couple several parts of the lance to each other, for example by threading means.

The lance is configured to engage a sample unit, preferably it is configured to be inserted into a sample unit. In other words, the immersion end of the lance carries a sample unit when used in a sampling system. In particular, the immersion tip is configured to engage a sample unit. The external diameter and shape of the immersion tip is configured to mate with a suitable sampling unit, especially to fit flush in a sample unit.

Preferably, the immersion end of the lance, in particular the immersion tip, has a tapered section, the tapered section being a section comprising a cross-section which is tapered in a longitudinal direction towards the tip of the immersion end. If not otherwise defined, the cross-sectional area is the cross-sectional area perpendicular to the longitudinal axis of the lance along its length. The immersion end, in particular the immersion tip, preferably has a frusto-conical-shape. Preferably, the diameter of the immersion end tapers by not more than 10% towards the immersion tip. The diameter of the immersion end may taper from a diameter between 10 to 40 mm towards a diameter between 5 to 38 mm. Such a shape allows the immersion lance to be press-fitted into a sample unit in an efficient way.

The immersion end may be part of the lance-body, it may also be provided as an additional part, in particular as an immersion tip. In preferred embodiments, the immersion tip of the lance is detachable. Thus, the immersion tip, which is the part of the lance which is exposed to the most wear in use, can be easily exchanged. The immersion tip may be fixated to the other parts of the lance by suitable means, for example by threading means of welding. The immersion tip can be foreseen with coupling means configured to couple the immersion tip to the adjacent part of the lance, for example by threading means. The immersion tip and the adjacent part of the lance thus may have respective internal and external diameters to enable the coupling. In other embodiments, the immersion tip and the adjacent part of the lance may have similar diameters, with their ends abutting flush to one another being joined by additional coupling means or a weld.

In preferred embodiments, the lance comprises protrusions located at the immersion end, preferably at the immersion tip of the lance. A protrusion is to be understood as a singular item that projects out of a surface. Such protrusions allow an easy but safe fixation of a sample unit. The protrusions may be part of the immersion end or the immersion tip, in other words the immersion end and the protrusions may be monolithically formed. The protrusions may also be provided as additional components. For example, the immersion end may be tube shaped and comprise a plurality of radial bores in the lateral surface of the tube. Protruding elements can be fixed in the bores, for example by threading means or welding. When the immersion end comprises a tapered section, the protrusions are preferably positioned within this tapered section.

The shape of the protrusions is not further restricted, they may for example be pyramidal, cylindrical, conical or frusto-conical shaped.

Preferably, the protrusions are provided in such a way that they cannot be pushed back when the immersion end is inserted into a sample unit, in particular, the protrusions are not springloaded. In preferred embodiments, the protrusions have a greater hardness than the face of the sample unit with which they engage.

The number of protrusions is not restricted, for example, the lance may comprise two, three, four, five, six or seven protrusions. Preferably, the protrusions are evenly distributed around the circumference of the immersion end.

In preferred embodiments, the lance comprises a collar at the immersion end. A collar is to be understood as a part which has a larger diameter than the rest of the lance and is arranged in such a way, that it protrudes from the axial length of the lance. Preferably, the collar is part of the immersion tip. In embodiments comprising protrusions, the collar is preferably positioned above the protrusions in a direction from the immersion end to the handling end.

Preferably, the collar is configured to sit flush on a sample unit. It has surprisingly been found that the provision of such a collar on the lance, which is to be directly placed on a sample unit, improves the usability of a sampling system for aluminum applications.

Preferably the outer diameter of the collar is at least 20 % larger than the outer diameter of the immersion end of the lance, more preferred at least 30 % larger, even more preferred at least 40% larger. In particular, the outer diameter of the collar should not exceed the diameter of the face of the sample unit on which it is arranged. The outer diameter of the collar is to be understood as the largest diameter of the collar perpendicular to the longitudinal axis of the lance, wherein the longitudinal axis is an axis extending between the immersion end to the handling end. In cases where the lance comprises a bend or a kink, the longitudinal axis is the axis extending on a straight portion between the immersion end to the bend.

Preferably, the side of the collar facing the immersion end of the lance is flat. Thus, the flat face forms a sealing face towards a sample unit when the lance is pressed in and on the sample unit. Further sealing means are not required which further improves the simple design of the sampling system and reduces the use of potentially contaminating materials.

The collar can have an inclined shaped face, in particular a conical shaped face, opposite the flat face. The collar may be ring shaped, it may also be frusto-conical shaped or a combination of these shapes.

The collar may be part of the immersion end, in other words the immersion end and the collar may be monolithically formed. The collar may also be provided as a separate component. In such cases, the collar may be part of the immersion tip of the lance which may also comprise protrusions.

The material of the collar may be the same or different as the material of the other components of the lance. Preferably, the collar is made from the same material as the immersion end and/or the immersion tip.

The lance may comprise additional parts arranged within a hollow body structure at the immersion end. Preferably, the lance comprises an inner tube arranged at the immersion end inside a hollow body structure of the lance. Such a tube can provide a ventilation path away from the sample unit when the unit is filled during sampling and allows to obtain samples with a higher quality.

The inner tube is preferably configured to be fixed inside the hollow body structure of the lance by suitable means, for example by threading means or welding.

A further aspect of the invention is a method for taking samples from a molten aluminum bath in a primary aluminum producing facility with a sampling system comprising a lance and a sample unit. The lance has an immersion end and a handling end. The sample unit is configured to receive a sample of molten aluminum and comprises an inlet opening and a sample chamber in flow connection with the inlet opening. The lance is configured to engage the sample unit. The method comprises the sequential steps
(a) providing the sampling system;
(b) immersing the sample unit and the immersion end of the lance in the molten aluminum bath;
(c) retracting the sample unit and the immersion end of the lance from the molten aluminum bath when a sample of molten aluminum has filled the sample chamber;
(d) separating the sample from the sample unit.

Surprisingly, it has been found that a sampling system comprising a lance and a sample unit can also be applied for aluminum sampling applications in primary aluminum producing facilities.

The sampling system to carry out the inventive method comprises a lance and a sample unit. All features, advantages and embodiments mentioned in relation to the sampling system for the lance and the sample unit according to the invention also apply to the inventive method.

In step (a), the method comprises providing the sampling system. Providing the sampling system is to be understood as providing a lance engaging a sample unit. In other word, the sampling system comprises a sample unit coupled on or to a lance.

In step (b), the method comprises immersing the sample unit and the immersion end of the lance in the molten aluminum bath. After step (b) the sample unit and the immersion end are positioned under the surface of the molten aluminum bath at a sampling position. During the immersion, the sample unit and the immersion end of the lance are moved through the cryolite layer which typically covers the molten aluminum bath.

To minimize the contact time of the immersion unit and the immersion end of the lance with the cryolite layer prior to the intake of the sample, the immersion speed needs to be high enough. Preferably, the immersion speed is at least 20 cm/s, more preferred at least 30 cm/s. For example, suitable immersion speeds are in the range of 20 - 50 cm/s.

During the passage of the sample unit and the immersion end of the lance through the cryolite layer, cryolite will freeze on the introduced cold parts when the materials of the lance and the sample unit are chosen in a suitable way. Thus, a crust of frozen cryolite will build up on the junction between the sample unit and the immersion end of the lance as well as on the outside of the sample unit. Surprisingly it has been found that this cryolite crust is favorable for the inventive method. It enhances the sealing of the junction as well as the sample unit, which allows the molten aluminum to enter the sample unit only through the inlet opening. Furthermore, the lance is protected from the molten aluminum as well as from ambient oxygen when retracted from the melt, which prolongs its lifetime and enhances its re-usability.

Additionally, the layer builds a protection of the lance against a shortcut current between the aluminum bath and the anode-blocks of the vessel when touched accidently.

The immersion of the sample unit and the immersion end of the lance may comprise more than one sub-step. For example, the step may comprise
(i) immersing the sample unit and the immersion end to a first position, in which the sample unit is in contact with the bottom of the molten aluminum bath and
(ii) retracting the sample unit and the immersion end to a sampling position between the bottom of the aluminum bath and the surface of the molten aluminum bath.

Such a sub-step procedure may ensure, that the sample unit is positioned at a suitable sampling position when the molten aluminum enters the sample chamber.

As soon as the immersion unit is positioned in the molten aluminum bath, the molten aluminum will flow into the sample chamber through the inlet opening. The molten aluminum which enters the sample chamber will build the sample. If means for covering the inlet opening are present on or at the sample unit, this filling will start after such means have dissolved, burned or otherwise disintegrated.

In step (c), the method comprises retracting the sample unit and the immersion end of the lance from the molten aluminum bath when a sample of molten aluminum has filled the sample chamber.

In step (d), the method comprises separating the sample unit from the sample. Thus, the sample is removed from the sampling system and in particular from the sample unit. The removal is typically conducted after a certain cooling period, during which the sample of molten aluminum solidifies in the sample chamber. It is to be understood, that removing the sample unit includes the destruction of the sample unit parts surrounding the sample.

Prior and/or after step (d) the method may comprise a step during which a cryolite crust is removed from the immersion end of the lance.

The method may comprise further steps. For example, the method my comprise a step during which the sample unit is removed from the lance and/or a step during which the remains of the sample unit are disposed in the molten aluminum bath.

Preferably, the sample is delivered to an analysis device after being removed from the sample unit. The sample can be directly analyzed by a suitable spectroscopic technique, for example by OES or LIBS spectroscopy.

The following schematic drawings show aspects of the invention for improving the understanding of the invention in connection with some exemplary illustrations, wherein
- Figure 1: shows a sampling system according to the invention in use.
- Figure 2: shows the immersion end of a lance with an attached sample unit in more detail.
- Figure 3: illustrates a schematic cross-sectional view on the immersion part of another exemplary inventive sampling system.

The drawings illustrate embodiments and together with the description serve to explain principles of the invention. The elements of the drawings are not necessarily to scale relative to each other. Like reference numerals designate corresponding similar parts.

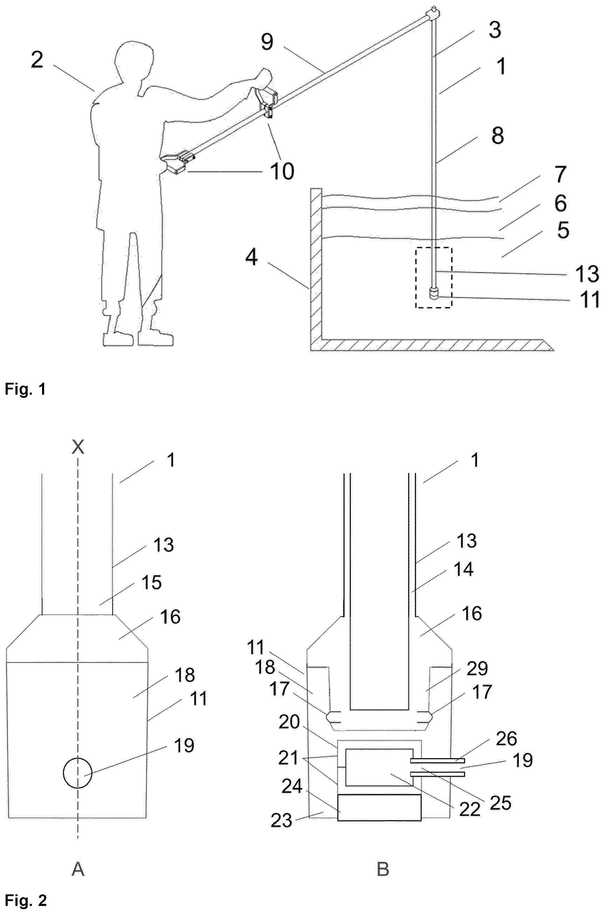

Figure 1 shows a sampling system 1 according to the invention in use. An operator 2 holds the lance 3 and inserts it manually in a vessel 4 containing a melt of aluminum 5. The aluminum bath 5 is covered with a layer of cryolite 6; a crust 7 covers the cryolite layer 6. The shown lance 3 comprises an immersion portion 8 and a handling portion 9 with handles 10. At the end of the immersion portion 8, i.e. the immersion end 13, a sample unit 11 is arranged.

For the sampling process, the operator 2 introduces the lance 3 into the vessel 4. During this process, the operator 2 has no visual contact to the interior of the vessel. The immersion end 13 holding the sampling unit 11 is immersed through the crust 7 and the cryolite layer 6 in the aluminum bath 5. The cryolite typically has a superheat of only 10 °C, i.e. its temperature exceeds the melting temperature of the material by only 10 °C. Thus, cryolite will freeze on the cold components of the sampling system 1 during the passage through the cryolite layer 6, in particular, it will freeze over the connection between the immersion end 13 and the sample unit 11. This frozen layer protects the lance 3, in particular its immersion end 13, from dissolving in the cryolite and also seals the joint between immersion end 13 and sample unit 11. After the immersion of the sample unit 11, the sample chamber 12 (not visible in Fig. 1) within the sample unit 11 will be filled by molten aluminum. After retraction of the immersion lance 3 and the sample unit 11 from the vessel 4, the sample unit 11 can be easily broken, releasing the sample.

After cooling, the cryolite frozen on the immersion lance becomes fragile and can be removed from the immersion lance, which can be re-used subsequently. The material of the lance, especially the immersion end 13, needs to be chosen to comply with the demands in the vessel and the corrosive nature of the cryolite material. The samples obtained with the inventive device 1 are directly available for an analysis, even at the place of sampling. All waste materials from the sample unit are selected to only comprise non-contaminating materials and can thus be disposed in the vessel with the molten aluminum, without generating waste or undesired additions in the molten metal.

Figure 2 shows the immersion end 13 of the lance 3 and the sample unit 1 of Fig. 1 (indicated by a dashed box) in more detail. In Fig. 2 A, a view on the exterior of the immersion end 13 of the lance 3 with a mounted sample unit 11 thereon is shown. The immersion end 13 comprises a part with a smaller diameter 15 and a part with a larger diameter, in this way forming a collar 16. On its outer circumference, the collar 16 sits flush on the sample unit 11. In the view of Fig. 2 A, only an outer refractory body 18 of the sample unit 11 is visible. A sideways arranged inlet 19 leads to a sample chamber assembly 20 arranged inside the refractory body 18 (not visible in this view).

The refractory material is a porous fireproof material, for instance an alumina-based sand. It has surprisingly been found that the use of a sand material with a low silicon content allows to use such a refractory body and a respective sample unit for alumina sampling applications. This attempt has previously not been considered due to the inherently high contamination of silica-based sand usually employed for steel sampling.

A 40 mm diameter sand body with a height of 45 mm comprises approximately 100 g sand, of which 47 g are pure silicon (Si) when a mixture containing 60 mass-% silica is used. An exemplary calculation shows, that in case only one sample is taken this will contribute to 45 ppm increase of Si in a standard Al bath when the remains of the sample unit are disposed into it after sampling. However, multiple samples are taken during a day and every single one would contribute additional to 45 ppm of Si. This can add up to 400 - 500 ppm a day, which is highly undesirable. To avoid such a high contamination, an alumina rich sand or pure alumina sand is the material of choice for aluminum sampling applications. For example, a 70 mass-% alumina sand will reduce the Si pickup with 70 %, reducing the Si pick-up to 12 - 15 ppm per sample.

In Fig. 2 B a schematic cross-section of the detailed view along line X in Fig. 2 A is shown. The sample unit 11 is engaged by the immersion end 13 of the lance. The lance comprises a main tube-shaped body 14 and an immersion end, which is designed with a collar 16 and protrusions 17.

The sample unit 11 has a conically shaped intake on the top side, in which the tip of the immersion end 13 is inserted. When the refractory sand body 18 is pushed on the immersion end tip so that the refractory body 18 surrounds the exterior of the immersion end tip, the protrusions 17 extending from the immersion end scratch the inner surface of the body - the sample unit 11 is thus held in place by these damages.

The sample chamber assembly 20 is arranged approximately axial symmetrically in the refractory body 18. It is constructed substantially cylindrically. The sample chamber assembly 20 comprises two half-shells 21 made of aluminum which surround the disc-shaped sample chamber 22. The height of the cylindrical sample chamber 22 bound by the parts of the half-shells 21 is substantially smaller than its diameter. For assembling the sample unit 11, the half shells 21 are inserted into an indentation of the refractory body 18 until they are sufficiently engaged by the refractory body 18. A very simple assembly is thereby possible. In the shown embodiment, the refractory body 18 comprises two parts: a main part 23 with a cylindrical shaped indentation, which comprises an inward-protruding collar und a closing bottom 24 which closes the bottom side after the sample chamber assembly 20 has been inserted in the indentation and been positioned on the collar.

At a side of the sample chamber assembly 20 an opening 25 is foreseen, which is arranged in flow communication with the inlet opening 19 of the surrounding refractory body 18 with an inflow conduit 26.

The sample chamber assembly 20 is located completely outside and apart from the immersion end 13 of the lance, which facilitates the releasing of the sample. It suffices to smash the refractory body 18 to free the sample from the sample unit 11.

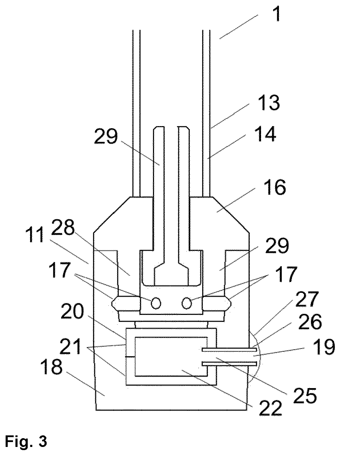

Fig. 3 shows another embodiment of the inventive sampling system in an analogue cross-sectional view as of Fig. 2 B. The inlet opening 19 is covered with a paper tape 27 in order to prevent the entering of cryolite into the sample chamber 12 before the sampling unit 11 is positioned in its final sampling position. The tape 27 will burn away easily during the immersion process.

The immersion end 13 of the lance has a modular design: it comprises a tip part 28 comprising the collar 16 and the protrusions 17, which can be welded to the main portion of the lance. In other words, the protrusions 17 are not an integral part of the immersion tip 28 but are separate parts which are fixated to the immersion tip 28.

Inside the tube 14 of the main body of the lance and extending through the immersion tip 28, an additional ventilation tube 29 is arranged, which bridges the connecting point between the sample chamber assembly 20 and the hollow immersion portion 8 of the lance. It has to be noted, that the lower part of this tube 29, which is ring shaped, is not shown in the cross-sectional plane as the other parts of the sampling system but protrudes from the paper plane towards the viewer. Such an additional ventilation tube 29 provides an enhanced ventilation path for gases escaping from the sample chamber 12 when it is filled by inflowing molten metal and allows to obtain samples with a higher quality in terms of homogeneity of the sample and especially its surface. The additional tube 29 can be connected to the immersion tip 29 by a threading mechanism: a threaded opening can be foreseen in the immersion tip 28 and the additional tube 29 can be externally threaded (not shown in the drawing). The fixation of such a ventilation tube 29 can also be foreseen in the tube 14 of the main body of the immersion end 13.

It is to be understood, that all described embodiments can also be present in combination if they do not contradict each other.

### Reference Signs

- 1: sampling system
- 2: operator
- 3: lance
- 4: vessel
- 5: aluminum bath
- 6: cryolite layer
- 7: crust
- 8: immersion portion of lance
- 9: handling portion of lance
- 10: handles
- 11: sample unit
- 12: sample chamber
- 13: immersion end
- 14: tube body of lance
- 15: part of immersion end with small diameter
- 16: collar
- 17: protrusions
- 18: refractory body
- 19: inlet
- 20: sample chamber assembly
- 21: half shells of sample chamber assembly
- 22: sample chamber
- 23: cylindrical main part of refractory body
- 24: closing bottom of refractory body
- 25: opening of sample chamber assembly
- 26: inflow conduit
- 27: paper tape
- 28: immersion tip
- 29: ventilation tube

## Claims

1. A sampling system for taking samples from a molten aluminum bath in a primary aluminum producing facility, comprising
- a lance, having an immersion end and a handling end,
- a sample unit configured to receive a sample of molten aluminum, comprising an inlet opening and a sample chamber in flow connection with the inlet opening,
wherein the lance is configured to engage the sample unit,
**characterized in that**
the immersion end of the lance is made from a non-magnetic material with a melting point higher than 950 °C.

2. A sampling system according to claim 1, wherein the immersion end of the lance is made from stainless steel.

3. A sampling system according to claim 1 or 2, wherein the immersion end of the lance has a tapered section.

4. A sampling system according to any of the preceding claims, wherein the immersion end of the lance is configured to be inserted into the sample unit.

5. A sampling system according to any of the preceding claims, wherein the lance comprises protrusions located at the immersion end.

6. A sampling system according to any of the preceding claims, wherein the lance comprises a collar at the immersion end.

7. A sampling system according to the preceding claim, wherein the collar is configured to sit flush on the sample unit.

8. A sampling system according to any of the preceding claims, wherein the sample unit comprises a top side and a bottom side and wherein the inlet opening is positioned in the face extending between the top side and the bottom side of the sample unit.

9. A sampling system according to any of the preceding claims, wherein the sample chamber has a circular cross-section perpendicular to the longitudinal axis of the sample unit

10. A sampling system according to any of the preceding claims, wherein the sample unit comprises a refractory body and a sample chamber assembly arranged within the refractory body.

11. A sampling system according to the preceding claim, wherein the refractory material of the refractory body has a silica content lower than 80 mass.-%.

12. A sampling system according to claim 10 or 11, wherein the material of the sample chamber assembly is copper or aluminum.

13. A sample unit for a sampling system according to claims 1 - 12.

14. A lance for a sampling system for taking samples from a molten aluminum bath in a primary aluminum producing facility,
wherein the lance has an immersion end and a handling end,
**characterized in that** the immersion end of the lance is made from a non-magnetic material with a melting point higher than 950 °C.

15. Method for taking samples from a molten aluminum bath in a primary aluminum producing facility, comprising the sequential steps
(a) providing a sampling system, comprising a lance and a sample unit,
wherein the lance has an immersion end and a handling end and
wherein the sample unit is configured to receive a sample of molten aluminum and comprises an inlet opening and a sample chamber in flow connection with the inlet opening and
wherein the lance is configured to engage the sample unit;
(b) immersing the sample unit and the immersion end of the lance in the molten aluminum bath;
(c) retracting the sample unit and the immersion end of the lance from the molten aluminum bath when a sample of molten aluminum has filled the sample chamber;
(d) separating the sample from the sample unit.
